# EUROPÄISCHE PATENTSCHRIFT

(11) **EP 2 792 389 B1**
(45) Veröffentlichungstag und Bekanntmachung des Hinweises auf die Patenterteilung: **18.09.2019**
(21) Anmeldenummer: 13164148.2
(22) Anmeldetag: 17.04.2013
(51) Int. Cl.: A61K 36/738, A61K 36/899, A61K 36/28, A61K 36/328, A61K 36/76, A61K 36/53, A61P 31/12, A61P 31/18

(54) **Antivirale Pharmazeutische Zusammensetzung, enthaltend einen Extrakt von Juncus acutus.**
Antiviral Pharmaceutical composition comprising an extract of Juncus acutus
Composition pharmaceutique antivirale à base d'un extrait de Juncus acutus

(43) Veröffentlichungstag der Anmeldung: 22.10.2014
(73) Patentinhaber: Global Health Solutions AG, 9495 Triesen (LI)
(72) Erfinder: Volkmar, Monica Herta, 9495 Triesen (LI)
(74) Vertreter: Hasler, Erich

(56) Entgegenhaltungen:
- WO-A1-98/42188
- WO-A1-02/094300
- US-A1- 2009 220 440
- DELLAGRECA M ET AL: "Benzocoumarins from the rhizomes of Juncus acutus", TETRAHEDRON, ELSEVIER SCIENCE PUBLISHERS, AMSTERDAM, NL, Bd. 59, Nr. 26, 23. Juni 2003 (2003-06-23) , Seiten 4821-4825, XP004431658, ISSN: 0040-4020, DOI: 10.1016/S0040-4020(03)00698-7
- DELLAGRECA M ET AL: "Dimeric phenanthrenoids from Juncus acutus", MEDICINAL & AROMATIC PLANTS ABSTRACTS, SCIENTIFIC PUBLISHERS, SCIENTIFIC PUBLISHERS, NEW DELHI - INDIA, Bd. 27, Nr. 5, 1. Oktober 2005 (2005-10-01), XP018005242, ISSN: 0250-4367
- AWAAD ET AL: "Phenolic glycosides of Juncus acutus and its anti-eczematic activity", MEDICINAL & AROMATIC PLANTS ABSTRACTS, SCIENTIFIC PUBLISHERS, SCIENTIFIC PUBLISHERS, NEW DELHI - INDIA, Bd. 28, Nr. 6, 1. Dezember 2006 (2006-12-01), XP018019818, ISSN: 0250-4367
- DELLAGRECA M ET AL: "New dimeric phenanthrenoids from the rhizomes of Juncus acutus. Structure determination and antialgal activity", TETRAHEDRON, ELSEVIER SCIENCE PUBLISHERS, AMSTERDAM, NL, Bd. 59, Nr. 13, 24. März 2003 (2003-03-24) , Seiten 2317-2324, XP004415520, ISSN: 0040-4020, DOI: 10.1016/S0040-4020(03)00237-0
- KOVACS ET AL: "Natural phenanthrenes and their biological activity", PHYTOCHEMISTRY, PERGAMON PRESS, GB, Bd. 69, Nr. 5, 19. Februar 2008 (2008-02-19), Seiten 1084-1110, XP022495588, ISSN: 0031-9422, DOI: 10.1016/J.PHYTOCHEM.2007.12.005
- NAHEED MAHMOOD ET AL: "The Anti-HIV Activity and Mechanisms of Action of Pure Compounds Isolated fromRosa damascena", BIOCHEMICAL AND BIOPHYSICAL RESEARCH COMMUNICATIONS, Bd. 229, Nr. 1, 1. Dezember 1996 (1996-12-01), Seiten 73-79, XP055075765, ISSN: 0006-291X, DOI: 10.1006/bbrc.1996.1759
- SUGANDA ET AL: "[Inhibitory effects of some crude and semi-purified extracts of indigenous French plants on the multiplication of human herpesvirus 1 and poliovirus 2 in cell culture].", JOURNAL OF NATURAL PRODUCTS, Bd. 46, Nr. 5, 1. Januar 1983 (1983-01-01) , Seiten 626-632, XP055075767, ISSN: 0163-3864
- YUCHAROEN RAENU ET AL: "Inhibitory effect of aromatic herbs, lavender, sage and chamomile against herpes simplex virus infection", AFRICAN JOURNAL OF BIOTECHNOLOGY, Bd. 10, Nr. 68, November 2011 (2011-11), Seiten 15394-15401, XP002712220, ISSN: 1684-5315
- KUSUMOTO I T ET AL: "SCREENING OF VARIOUS PLANT EXTRACTS USED IN AYURVEDIC MEDICINE FOR INHIBITORY EFFECTS ON HUMAN IMMUNODEFICIENCY VIRUS TYPE 1 (HIV-1) PROTEASE", PHYTOTHERAPY RESEARCH, JOHN WILEY & SONS LTD. CHICHESTER, GB, Bd. 9, Nr. 3, 1. Mai 1995 (1995-05-01), Seiten 180-184, XP001087900, ISSN: 0951-418X
- MARJORIE MURPHY COWAN: "Plant Products as Antimicrobial Agents", CLINICAL MICROBIOLOGY REVIEWS, WASHINGTON, DC, US, Bd. 12, Nr. 4, 1. Oktober 1999 (1999-10-01), Seiten 564-582, XP007920538, ISSN: 0893-8512
- MUKHTAR M ET AL: "Antiviral potentials of medicinal plants", VIRUS RESEARCH, AMSTERDAM, NL, vol. 131, no. 2, 1 February 2008 (2008-02-01), pages 111-120, XP026862992, ISSN: 0168-1702 [retrieved on 2007-11-05]

## Beschreibung

Die Erfindung betrifft eine pharmazeutische Zusammensetzung, insbesondere zur Verwendung als antivirale Zusammensetzung oder Virostatikum gemäss Oberbegriff von Anspruch 1.

### Stand der Technik:

Lavandula angustifolia (zu deutsch: Lavendel; in der Literatur verwendete Synonyme: Lavandula officinalis, Lavandula vera) ist seit altersher als Heilpflanze bekannt. Die ätherischen Öle haben eine entzündungshemmende, analgetische und antimikrobielle Wirkung. Darüber hinaus kann die Pflanze das Abheilen von Wunden beschleunigen. In der US 2009/0220440 A1 ist eine Zusammensetzung für die topische Behandlung von Herpes offenbart, welche u.a. ätherische Öle der Melissa officinalis und Lavandula angustifolia enthält. Die Lavandula angustifolia wird dabei wegen der ihr zugeschriebenen Eigenschaften (siehe oben) und nicht wegen eines antiviralen Effekts eingesetzt. In Kombination sorgt die Zusammensetzung für ein schnelleres Abheilen der durch einen Herpes-Ausbruch verursachten Wunden.

Commiphora molmol (zu deutsch: Myrrhe) ist als vorwiegend antiseptisches und entzündungshemmendes Mittel zur äusserlichen Behandlung von Mund- und Racheninfekten (Zahnfleischentzündung und andere Zahnfleischerkrankungen, Mandelentzündung, Mundgeschwüre) bekannt. Die bitter schmeckende und adstringierende Myrrhe wird traditionell bei zahlreichen Beschwerden genutzt, wie bei Erkältungen (Erleichterung von Nasenatmung und Abhusten), Wunden und Geschwüren, insbesondere Infekten von Mund, Zahnfleisch und Rachen.

Myrrhe ist eine aromatische gummiartige Substanz, die von bestimmten Bäumen und Sträuchern, hauptsächlich der Familie der Burseraceae, die in Afrika und im mittleren Osten wachsen, abgesondert wird. Meistens wird Myrrhe als Harz gesammelt, das von dem Stamm des Myrrhe- Gummibaums Commiphora myrrha, Commiphora abyssinica oder Balsamodendron myrrha produziert wird.

Chamomilla matricaria (in der Literatur auch als Matricaria recutita und Chamomilla recutita bezeichnet; zu Deutsch: Kamille) ist eine Pflanzenart in der Familie der Korbblütler (Asteraceae). Sie ist eine alte Heilpflanze, die vor allem bei Magen- und Darmbeschwerden und bei Entzündungen Verwendung findet.

Die Stechende Binse (Juncus acutus) ist eine halophytische Pflanzenart aus der, Familie der Binsengewächse (Juncaceae).

Das Extrakt der Rosa damascena (eine Rosenart (Zuchtform)), insbesondere das Rosenöl, ist bekannt für seine krampflösenden, antiviralen und antiseptischen therapeutischen Eigenschaften. Es ist ausserdem ein gutes Bakterizid. Es kann bei der Behandlung von Typhus, Durchfall, Cholera, Lebensmittelvergiftungen und andere Krankheiten, die durch Bakterien verursacht sind, verwendet werden. Weiterhin heilt es interne bakterielle Infektionen z.B. im Dickdarm, Magen, Darm und Harnwegen, sowie externe Infektionen auf der Haut, Ohren, Augen und in Wunden.

Die Silberweide (Salix alba) ist ein Laubbaum aus der Gattung der Weiden (Salix), welche zur Familie der Weidengewächse (Salicaceae) gehört. Für Weidenrinde sind antipyretische, antiphlogistische und analgetische Eigenschaften bekannt.

Virusinfektionen sind mit Recht die widerstandsfähigsten von allen bekannten Erkrankungen mit infektiöser Krankheitsursache mit denen man in der klinischen Medizin zu kämpfen hat. Obwohl verschiedene Prophylaxeprogramme gegen Virusinfektionen sehr erfolgreich waren, z. B. Impfung gegen Polio, und in einem geringeren Ausmaß gegen Grippe, gibt es sehr wenige Erfolgsberichte im Zusammenhang mit der Entwicklung akuter Heilungstherapien oder antiviraler Antibiotika. Tatsächlich sind Nukleosidanaloga, wie Acycloguanosin, die bei der Bekämpfung von Infektionen mit Herpes simplex I und II wirksam sind, fast die einzigen bekannten antiviralen Mittel.

In die Entwicklung wirksamer Mittel und Prophylaktika für Virusinfektionen wurde bisher viel Geld investiert. Allerdings liessen die Resultate zu wünschen übrig. Weil viele Virusinfektionen sehr verbreitet sind und zum Teil grosse wirtschaftliche Schäden verursachen, besteht ein grosser Bedarf nach wirksamen und relativ preiswerten Prophylaktika und Mitteln für die Bekämpfung von Virusinfektionen.

Das HIV- Virus gehört zu den Retroviren, die man auch als Leukämieviren bzw. RNA-Tumorviren bezeichnet hat. Im Unterschied zu anderen Retroviren weist das HIV- Virus keine Fähigkeit zur Zelltransformation auf. Der tödliche Ausgang der Aids-Krankheit beruht nicht auf einer Wirkung als Tumorvirus, sondern auf der Lyse von Helfer-T-Zellen.

Obwohl in den vergangenen Jahren Fortschritte in der Prävention und Behandlung von AIDS gemacht wurden, mangelt es immer noch an Medikamenten, welche effektiv sind und keine oder nur geringe Nebenreaktionen auslösen.

### Aufgabe

Es besteht deshalb ein grosses Bedürfnis nach der Bereitstellung eines gut verträglichen Mittels zur Bekämpfung diverser viraler Infektionskrankheiten, insbesondere von Herpes labialis und Herpes zoster Infektionen, sowie von HIV-Erkrankungen. Es ist insbesondere ein Ziel der vorliegenden Erfindung, eine auf natürlichen Stoffen basierende Zubereitung oder Zusammensetzung vorzuschlagen, mit welcher Viren, insbesondere Grippeviren, Herpes- und HIV-Viren, bekämpft werden können. Noch ein Ziel ist es, eine Zusammensetzung oder Zubereitung vorzuschlagen, welche keine oder nur geringe Nebenwirkungen hat.

### Beschreibung

Diese und weitere Ziele werden durch den Gegenstand gemäss Anspruch 1 erreicht. Vorteilhafte Ausgestaltungen des erfindungsgemässen Gegenstands sind in den Unteransprüchen definiert.

Gemäß einem Aspekt stellt die vorliegende Erfindung daher eine antivirale Zusammensetzung, d.h. eine Zusammensetzung für die Vorbeugung und/oder Behandlung oder Bekämpfung von Virusinfektionen oder Viruserkrankungen bei einem Menschen oder einem Säugetier zur Verfügung.

Der Begriff "Bekämpfen" umfaßt, wie er hier verwendet wird, sowohl eine Prophylaxe als auch eine Therapie, d. h. sowohl das Vorbeugen als auch das Behandeln von Virusinfektionen oder Viruserkrankungen, einschließlich einer Erleichterung oder Linderung von Symptomen, oder eines Anhaltens oder einer Verlangsamung des Fortschreitens oder der Entwicklung der Erkrankung oder Infektion. Der Begriff "antiviral" schließt alle Formen der Wirkung gegen den Virus ein, einschließlich einer Aktivität bei der Vorbeugung oder Verminderung der Infektion durch den Virus, oder der Virusübertragung in den Wirt, des Überlebens des Virus oder der Virenvermehrung usw.

Überraschend wurde festgestellt, daß eine Zusammensetzung, die ein Extrakt der Pflanzenart Stechende Binse (Juncus acutus) und weiteren Pflanzenart aus der Gruppe von Silberweide, insbesondere Salix alba, Rose, insbesondere Rosa damascena, Kamille, insbesondere Chamomilla matricaria, Lavendel, insbesondere Lavandula angustifolia und Myrrhe, insbesondere Commiphora molmol als Inhaltsstoffe aufweist, bei der Vorbeugung von Virusinfektionen oder der Behandlung oder Linderung der Symptome von Virusinfektionen wirksam ist.

Diese Feststellung ist bemerkenswert, da ein Extrakt der Stechende Binse und Extrakte der Rose, Kamille, Lavendel, Silberweide, und Myrrhe, wie vorstehend dargelegt, schon seit langer Zeit vom Menschen in großem Umfang genutzt wurden, und obwohl bekannt ist, daß die vorgenannten Heilpflanzen unterschiedliche medizinische Eigenschaften besitzen. So wird beispielsweise Rosen-, Kamillen- und Lavendelextrakten u.a. auch eine antivirale Wirkung nachgesagt, obwohl diese nicht zu deren Hauptanwendung zählt. Es wurde bislang jedoch niemals ein Hinweis darauf gegeben oder ein Vorschlag gemacht, daß eine erfindungsgemässe Kombination, die diese Extrakte aufweist, bei der Behandlung und/oder Vorbeugung von Virusinfektionen oder Viruserkrankungen bei Menschen oder anderen Tieren nützlich sein könnte.

Sowohl Extrakte der Stechenden Binse als auch Rosen-, Kamillen-, Lavendel-, Silberweide- und Myrrhe-Extrakte sind bekannt und im Handel erhältlich. Diese Extrakte sind in verschiedenen Formen erhältlich, z. B. als Harze, Tinkturen oder andere Lösungen, Pulver. Diese können aus der Rinde, dem Harz, dem Kraut, den Blütenständen der Pflanze gewonnen werden. Geeignete Quellen oder Zubereitungen der vorgenannten Pflanzen sind ausführlich in der Literatur beschrieben oder handelsüblich erhältlich. So werden zur Herstellung des Extraktes die Blüten der Kamille, Rose und Lavendel, das Baumharz der Myrrhe, die Rinde der Silberweide und das Gras der stechenden Binse verwendet. Vorzugsweise findet dabei die Extraktion mit 96%-igem Alkohol bei Raumtemperatur statt (Mazeration).

Extrakte der Stechende Binse als auch der Rose, Kamille, Lavendel, Silberweide und Myrrhe aus den geeigneten Pflanzenteilen oder Pflanzenabsonderungen (z. B. Myrrhe-Harz) können unter Verwendung eines oder mehrerer Lösungsmittel, wie Wasser, Ethanol, Methanol, Hexan, Ether, Aceton, Ethylacetat, Toluol, Benzol, Propylenglycol, Glycerin und dergleichen oder einer Mischung davon hergestellt werden. In Abhängigkeit von dem Zustand der Extraktfraktion oder Zubereitung kann ein Teil der Extraktfraktion beispielsweise von dem Lösungsmittel durch Verdampfen getrennt - werden und dann direkt als Extrakt mit antiviralen Eigenschaften eingesetzt werden.

In der vorliegenden Erfindung können auch Derivate und Analoga der Stechenden Binse, der Rose, Kamille, Lavendel, Silberweide und Myrrhe verwendet werden. Diese werden im Rahmen der vorliegenden Erfindung unter die allgemeinen Begriffe "Stechende Binse", "Rose", "Kamille", "Lavendel", "Silberweide" und "Myrrhe" subsumiert.

Vorteilhaft umfasst die Pflanzengattung Lavendel die Pflanzenarten Lavandula officinalis, Lavandula stoechas, Lavandula dentata, Lavandula latifolia. Weiter kann die Pflanzengattung Rose die Pflanzenarten Rosa canina, Rosa chinensis, Rosa hybrida, Rosa manetti, Rosa nitida und Rosa multiflora umfassen.

Jedes beliebige der im Stand der Technik zur Gewinnung von Extrakten der Stechenden Binse, der Rose, Kamille, Lavendel, Silberweide und Myrrhe beschriebenen Verfahren kann zur Durchführung der vorliegenden Erfindung eingesetzt werden. Tinkturen (d. h. alkoholische oder wäßrig-alkoholische Lösungen) der Stechenden Binse, der Rose, Kamille, Lavendel, Silberweide und Myrrhe sind handelsübliche Produkte, die zweckmäßigerweise erfindungsgemäß verwendet werden können.

Die aktiven Inhaltsstoffe können mit einem oder mehreren üblichen Trägern, Lösungsmitteln, Verdünnungsmitteln und/oder Grundstoffen vorliegen, um übliche Zubereitungen, wie Tabletten, Pillen, Pulver, Pastillen, Kissen, Kapseln, Elixiere, Suspensionen, Emulsionen, Lösungen, Sirupe, Aerosole (als Feststoff oder in einem flüssigen Medium), Salben, Weich- und Hartgelatinekapseln, Zubereitungen für örtliche Anwendung, steril verpackte Pulver, Mundwässer oder Mundspülungen und dergleichen herzustellen.

Beispiele für geeignete Träger, Grundstoffe und Verdünnungsmittel sind Lactose, Glucose, Saccharose, Sorbitol, Mannitol, Stärken, Akaziengummi, Calciumphosphat, Alginate, Traganth, Gelatine, Calciumsilicat, mikrokristalline Cellulose, Polyvinylpyrrolidon, Cellulose, Wassersirup, Wasser, Wasser/Ethanol, Ethanol, Wasser/Glycol, Wasser/Polyethylen, Glycol, Propylenglycol, Methylcellulose, Methylhydroxybenzoate, Propylhydroxybenzoate, Talkum, Magnesiumstearat, Mineralöl oder fettartige Substanzen, wie Hartfett, oder geeignete Mischungen daraus. Die Zusammensetzungen können zusätzlich Gleit- bzw. Schmiermittel, Netzmittel, Emulgatoren, Suspensionsmittel, Konservierungsstoffe, Süßstoffe, Aroma- bzw. Geschmacksstoffe und dergleichen enthalten. Das Medikament kann so formuliert sein, daß es nach Verabreichung an den Patienten eine schnelle, anhaltende oder verzögerte Freisetzung der aktiven Inhaltsstoffe bereitstellt.

Im Rahmen der vorliegenden Erfindung werden insbesondere Extrakte der Pflanzenart "Juncus acutus", von den Extrakten der Pflanzengattung "Silberweide" insbesondere Extrakte der Pflanzenart "Salix alba", von den Extrakten der Pflanzengattung "Rose" insbesondere Extrakte der Pflanzenart "Rosa damascena", von den Extrakten der Pflanzengattung "Kamiiie" insbesondere Extrakte der Pflanzenart "Chamomilla matricaria", von den Extrakten der Pflanzengattung "Lavendel" insbesondere Extrakte der Pflanzenart "Lavandula angustifolia" und von den Extrakten der Pflanzengattung "Myrrhe" insbesondere Extrakte der Pflanzenart "Commiphora molmol" bevorzugt. Extrakte der vorgenannten Pflanzenarten haben sich als besonders vorteilhaft und wirksam bei der Behandlung von viralen Erkrankungen und Infektionen erwiesen, wenn die vorgenannten Extrakte zusammen eingesetzt werden.

Nachfolgend werden Ausführungsbeispiele der Erfindung anhand von durchgeführten Versuchen und Studien näher charakterisiert.

Produktion von VSV-pseudotypisierten und HIV-1 rekombinanten Viren Hochtitrige VSV-pseudotypisierte oder HIV-1 rekombinante Virenbestände wurden in 293T-Zellen durch Kotransfektion von pNL4-3.Luc.R-E- produziert, wobei entweder das pcDNA3-VSV oder das epNL3-Plasmid das G-Protein des vesikulären Stomatitis-Virus bzw. das HIV-1-Envelope-Gen unter Verwendung des Calciumphosphat-Transfektionssystems kodiert. Überstände, die Virusbestände enthalten, wurden 48 Stunden nach der Transfektion entnommen und 5 Minuten bei 500 g zentrifugiert, um die Zelltrümmer zu entfernen, und bis zur Verwendung bei -80 °C gelagert. Zellfreie Virusbestände wurden unter Verwendung eines Enzymimmunoassays zum Nachweis des HIV-Antigens (p24) getestet und Kulturen wurden bei einer Dosis von 200 ng HIV-1-Gag-Protein (p24) getestet.

### VSV-pseudotypisierter HIV-1-Infektionsassay

Epitheliale A549-Lungenzellen (106/ml) wurden auf einer 24-Lochplatte platziert und 30 Minuten lang mit den Testsubstanzen vorbehandelt. Nach der Vorbehandlung wurden die Zellen mit Virusbeständen (200 ng p24) inokuliert und 12 Stunden später wurden die Zellen zweimal in PBS gewaschen und über einen Zeitraum von 15 min bei RT in 25 mM Tris-Phosphat pH 7,8, 8 mM MgCl2, 1 mM DTT, 1 % Triton X-100 und 7 % Glyzerin lysiert. Dann wurden die Lysate der Spin-Down Technologie unterzogen und die Überstände wurden verwendet, um die Luciferase-Aktivität unter Verwendung eines AutoLumat LB 9510 (Berthold, Bad Wildbad, Deutschland) gemäß den Instruktionen des Luciferase-Assay-Kits (Promega) zu messen. Die Ergebnisse werden als % der Infektion dargestellt (unter Berücksichtigung der infizierten und unbehandelten Zellen 100 % Infektion). Die Ergebnisse stellen den Mittelwert ± SD 3 verschiedener Experimente dar. Bei einer anderen Reihe von Experimenten wurden die Zellen infiziert und 30 Minuten später mit der Testsubstanz behandelt.

In den nachfolgend beschriebenen Versuchen wurde jeweils eine Zusammensetzung enthaltend Extrakte des Juncus acutus, der Salix alba, Rosa damascena, Chamomilla matricaria, Lavandula angustifolia und der Commiphora molmol eingesetzt und zwar hergestellt wie unter Versuch 7 unten beschrieben.

### Versuch 1: Antivirale Aktivität der Zusammensetzung und Wirkmechanismus

Die anti-HIV-Aktivität der Zusammensetzung wurde anhand des Einflusses auf die Virusgentranskription gemessen. Dabei stellte es sich heraus, dass die Zusammensetzung einen anderen Wirkmechanismus als die klassischen antiviralen Pharmaka aufweist: Letztere wirken durch Hemmung der T-Zell-Aktivierung auch immunsuppressiv (was unerwünscht ist), während die vorliegende Zusammensetzung keine immunsupprimiernden Effekte zeigte.

Aussagen zur Immunsuppression konnten abgeleitet werden aus der Testung an HIV-Infizierten Jurkat-Zellen im Modell der HIV-LTR-Gentranskription unter T-Aktivierung durch CD3/CD8-Immunzellen. Es ist bekannt, dass antivirale Arzneimittel in der Regel die unerwünschte Nebenwirkung haben, die T-Zell-Aktivierung zu hemmen. Die erfindungsgemässe Zusammensetzung weist diesen für die Therapie unerwünschten Hemmeffekt jedoch nicht auf.

Wirkungen der Zusammensetzung auf die Virusvermehrung in HIV-infizierten Zellen im Modell der VSV-pseudotypisierten Lentivirusinfektion (es handelt sich hier um ein HIV-Modell, das die normalen Schritte des Einschleusens des Virus in die Zelle umgeht, und damit eine bessere Beurteilung des Angriffspunktes zulässt.

### Versuch 2: LTR-Aktivierung

Bei Jurkat-LTR-GFP handelt es sich um einen aus Jurkat-Zellen gewonnenen Klon, der latent mit einem rekombinanten Virus infiziert ist, das das durch den HIV-LTR-Promoter gesteuerte GFP-Gen enthält [Márquez N, Calzado MA, Sänchez-Duffhues G, Perez M, Minassi A, Pagani A, Appendino G, Diaz L, Muñoz-Fernández MA, Muñoz E. (2008), Differential effects of phorbol-13-monoesters on human immunodeficiency virus reactivation (Differentialwirkungen von Phorbol-13-Monoestern auf die Reaktivierung des Humanen Immundefizienz-Virus). Biochem Pharmacol. 75:1370-80]. Jurkat-LAT-GFP-Zellen exprimieren CD3 und CD28 und können mit mAbs Anti-CD3 und Anti-CD28 stimuliert werden. Die Zellen wurden 12 Stunden stimuliert und die Expression von GFPwurde durch Durchflusszytometrie in einem Durchflusszytometer vom Typ FACSCanto II analysiert. Es wurden zehntausend Ereignisse pro Probe gezählt und das Fluoreszenzmuster wurde bestimmt.

Bei Vorbehandlung der Zellen vor Infektion wird die Rate der Virusvermehrung in der Zelle gesenkt. Die Zusammensetzung hemmt die Transkription der Virusgene, und damit die Biosynthese neuer Viren.

Aufbauend auf diesen Vorversuchen wurden Experimente mit einer alkoholreduzierten Form der Zusammensetzung durchgeführt, um den Mechanismus der Hemmung der HIV-1-Replikation weiter einzuengen.

### Versuch 3: Effekt auf die VSV-pseudotypisierte HIV-Replikation in Lungenepithelzellen

Bei Vorbehandlung mit einer 0,5%igen Verdünnung der Zubereitung (= 0.5%-ige alkoholische Lösung des Konzentrats von Versuch 7) wurde die Infektion der Zellen vollständig verhindert. Wurden die Zellen erst infiziert und dann mit der Zusammensetzung behandelt, zeigte sich dennoch ein Hemmeffekt auf die Virustranskription: Wieder war die Hemmung vollständig bei einer Konzentration von 0,5% der Zubereitung.

Bei den verschiedenen Versuchen wurde 0.001 bis 1% w/w, vorzugsweise 0.01 bis 0.1 w/w wässerige Lösungen des alkoholischen Extrakts für die Inkubation der Zellen verwendet.

### Versuch 4: Effekt auf die durch TNF-α induzierte Virusreplikation in HeLa-Zellen:

Es wurde ein etabliertes Modell der HIV-1-Latenz und der aktiven Replikation auf Grundlage der Infektion der HeLa-Zellen bei geringer Multiplizität der Infektion (MOI) mit HIV-gewonnenen Partikeln verwendet, bei denen das Minigenom in Plasmid pEV731 (LTR-Tat-IRES-GFP-LTR) kodiert wird. Die Zellen wurden durch GFP-Expression sortiert und die HeLa-GFP-Zellen stellen latent mit HIV-1 infizierte Zellen dar, die durch externe Stimuli wie Zytokine oder Phorbolester reaktiviert werden können. HeLa-GFP+-Zellen weisen eine produktive HIV-1-Infektion auf. Die Zellen wurden 12 Stunden lang behandelt und die GFP-Expression wurde durch Durchflusszytometrie in einem Durchflusszytometer vom Typ FACSCanto II analysiert. Es wurden zehntausend Ereignisse pro Probe gezählt und das Fluoreszenzmuster wurde bestimmt. Um die potentielle Aktivität der Testsubstanz auf der HIV-LTR-GenTranskription zu testen, wurden die HeLa-LAT-GFP-Zellen verwendet, die ein einzelnes vollständiges, integriertes HIV-Provirus enthalten, in dem, GFP durch Nef ersetzt wurde. Diese Substitution ermöglicht eine schnelle Auswertung der Transkriptionsaktivität des HIV durch durchflusszytometrischen Nachweis der GFP-Epifluoreszenz, wenn externe Agonisten wie PKC-Aktivatoren oder Zytokine die Zellen aktivieren. Die Zellen wurden mit steigender Konzentration der Zusammensetzung vorinkubiert und anschließend über einen Zeitraum von 12 Stunden mit TNFa (10 ng/ml) stimuliert.

Versuche zeigten, dass eine 0,5%-ige alkoholreduzierte Form der Zusammensetzung (dabei wird die alkoholische Lösung, welche nach der Extraktion zwischen 80% und 93% Alkohol, Rest Wasser enthält, mit Wasser so weit verdünnt, dass eine 0,5%-ige alkoholreduzierte Form gebildet ist) die Transaktivierung des HIV-Virus um 50% hemmt, oder noch präziser, die Virusreplikation und die Transkriptionsaktivität am HIV-LTR-Promoter hemmt. Demnach stellt die Zusammensetzung einen potenten Hemmstoff sowohl des Einbaus von HIV-1 in das Genom der Wirtszelle, als auch der Virusvermehrung dar. Die Aktivierung von NF-kappa B (= NPκB) ist einer der Mechanismen, der zur Ablesung der Virusgene und damit zur Virusreplikation führt. Dass HIV-1 ins Genom eingebaut wird, konnte aus den Versuchen an A549-Zellen mit dem Luciferase-Assay gefolgert werden, wo sich ein Effekt der Präinkubation zeigt.

Die Vorbehandlung der Zellen 30 Min. vor der Infektion mit zunehmender Dosis der Zusammensetzung führte zu einer dosierungsabhängigen Hemmung der Luciferase-Aktivität. Interessanterweise stellte sich heraus, dass die hemmende Aktivität der Zusammensetzung verschwand, als die Zellen infiziert wurden und die Testsubstanz 30 Minuten später hinzugegeben wurde. Dies legt nahe, dass die hemmende Aktivität durch das Abzielen auf frühere Schritte im Lebenszyklus des Virus (d. h. reverse Transkriptase oder virale Integration) aufgehoben wurde.

TNFα wurde Mitte der 70er Jahre als ein durch Endotoxin induzierbarer Serum-Faktor entdeckt. Der Name des Proteins leitet sich von seiner Fähigkeit ab, im Tierexperiment Zellen von transplantierten Tumoren zu nekrotisieren sowie den Zelluntergang von neoplastischen Zelllinien bei *in vitro* Experimenten hervorzurufen. Humaner TNFα wird initial als 233 Aminosäuren (AS) langes transmembranäres Protein synthetisiert und kann anschließend als ein 157 Aminosäuren langes, nicht glykosyliertes Protein von der Zellmembran abgespalten werden.

TNFα spielt eine Schlüsselrolle im Start und Ablauf einer Entzündung. Bei der Ausschaltung mikrobieller Erreger und Viren führt dieses Zytokin zur Einwanderung und Aktivierung von neutrophilen Granulozyten und Monozyten am Ort der Infektion. Neben diesen lokalen Wirkungen kann TNFα systemische Effekte wie Fieber und Kachexie, sowie die Synthese von Akut-Phase-Proteinen der Leber und Aktivierung von Lymphozyten hervorrufen. TNFalpha ist wiederum ein Induktor von NF-kappaB, das bei Aktivierung für ein Ablesen der Virusgene sorgt (siehe nachfolgende Beschreibung).

### Versuch 5: Wirkungsmechanismus der Zusammensetzung

Stimulation infizierter Zellen mit TNF-α löst die Ablesung der Virusgene und damit die Virusvermehrung aus. Diese Aktivierung wird mit einer 0,2 prozentigen Lösung der Zubereitung halbmaximal gehemmt.

Diese Hemmung findet nicht auf der Virusgenebene statt, sondern konnte in Jurkat-Zellen auf eine Hemmung der Aktivierung von NF-κB (=nuclear factor 'kappa-lightchain-enhancer' of activated B-cells) in der Zelle zurückgeführt werden, und damit auf einen Mechanismus oberhalb der Ebene der Virusgene - vermutlich durch Hemmung einer oder mehrerer Kinasen, die - ausgelöst durch TNF-α, ihrerseits NF-κB aktivieren, was dann wiederum zur Ablesung einer ganzen Genfamilie führt, unter anderem auch von den Genen von HIV. Die Hemmung dieser NF-κBaktivierenden Kinase durch die Zusammensetzung führt damit indirekt zu einer Hemmung der Ablesung des Virusgens.

### Versuch 6: immunmodulierender Effekte der Zubereitung in humanen T-Zellen,des Immunsystems

Eine weitere Versuchsserie diente der Untersuchung immunmodulierender Effekte der Zubereitung in humanen T-Zellen des Immunsystems. Die Zubereitung hemmte dosisabhängig die stimulierte Aktivierung von T-Zellen. Die Hemmung von NF-κB spiegelt sich darin, dass in diesem Versuch die durch dieses System aktivierten Oberflächermarker CD69 reduziert wurden

### Durchflusszytometer-Analysen der Antigen-Expression der Zelloberfläche (Methode für die Oberflächenmarker)

Periphere mononukleäre Blutzellen wurden über die angegebenen Zeiträume mit SEB stimuliert, wobei die Konzentrationen der Zusammensetzung erhöht bzw. nicht erhöht wurden und die Oberflächen-Expression von CD25 (72 h), CD69 (24 h) Antigenen wurde mit spezifischem mAbs durch Durchflusszytometrie analysiert. CD25 ist die Kette des Interleukin-2-Rezeptors mit geringer Affinität und wird auf aktivierten T- und B-Zellen und aktivierten Makrophagen exprimiert. CD25 wurde mit dem mAb R0811 (PE-konjugiert) des Dako (Klon ACT-1) ermittelt. Die ruhenden T-Zellen exprimieren kein CD69, dieses wird jedoch durch In-Vitro-Aktivierung der T-Zellen hochreguliert. CD69 ist ein Frühaktivierungs-Antigen, das vor CD25 und CD71 exprimiert wird. Die Exprimierung von CD69 wurde durch ein FITC-markiertes mAb (Klon FN50) von BD Pharmigen festgestellt.

Die Oberflächenmarker werden bei Aktivierung der ruhenden infizierten Zelle exprimiert. Die Exprimierung von CD69 steht im Zusammenhang mit einem NF-kappaB-gesteuerten Mechanismus. Es ist also zu erwarten, dass eine Substanz, die NF-kappaB hemmt, auch die Bildung des Oberflächenmarkers CD69 hemmt.

### Versuch 7: Anwendung der Zubereitung auf Zellkulturen

Für die Herstellung von 200 Litern der antiviralen Zusammensetzung (entsprechend ungefähr 200 Kg) wurden folgende Mengen Ausgangsprodukt (jeweils Trockenware) mit reinem Ethanol 96 % (Rest gereinigtes Wasser) extrahiert:

| | |
|---|---|
| Kamillenblüten (Blütenstand mit Korb und Blüte) | 4.00 Kg |
| Rosenblüten (Rosa damascena) | 8.00 Kg |
| Lavendelblüten | 1.20 Kg |
| Myrrhe (Harz) | 0.07 Kg |
| Binsengras (Juncus acutus) | 3.00 Kg |
| Weidenrinde | 0.80Kg |

Nach der Extraktion (Mazeration) des Extraktionsgutes wurde sank der Alkoholgehalt durch die Aufnahme von Restwasser aus den getrockneten Pflanzen auf unter 90% ab. Dieses alkoholische Extrakt wurde zur Anwendung beim Menschen mit Wasser auf 0,1 bis 1,0 % Alkoholgehalt verdünnt.

Es hat sich gezeigt, dass die Wirksamkeit des Extrakts als antivirales Mittel nicht beeinträchtig wird, wenn die Extraktion mit einer wässerigen Alkoholmischung erfolgt, die wenigstens 80% Ethanol enthält. Bevorzugt erfolgt die Extraktion mit 90 bis 96 %-igem Ethanol (Rest Wasser).

Die Einzelextrakte wurden im Labor unter Imitierung der Bedingungen des Gesamtextraktes hergestellt (Ethanol 90% v/v) als Auszugsmittel. Für jeden Einzelextrakt wurde die anteilige Menge der jeweiligen Pflanze extrahiert, wie sie auch in der Zusammensetzung enthalten ist.

Getestet wurde im Modell der TNFalpha und/oder PMA-induzierten HIV-1-Aktivierung in infizierten Jurkat-Zellen.

### Methode:

Bei Jurkat-LTR-GFP handelt es sich um einen aus Jurkat-Zellen gewonnenen Klon, der latent mit einem rekombinanten Virus infiziert ist, das das durch den HIV-LTR-Promoter gesteuerte GFP-Gen enthält. Jurkat-LAT-GFP-Zellen reagieren auf TNFa und PMA, das den kanonischen Pfad der NF-kB-Aktivierung aktiviert.

Zuerst wurden die Zellen mit steigenden Konzentrationen der einzelnen, pflanzlichen Stoffe (vol/vol) vorinkubiert, um die Zytotoxizität zu bestimmen. Die Zytotoxizitätsassays wurden durch Einfärben von Propidiumiodid und Messung der toten Zellen per Durchflusszytometer durchgeführt. Die Konzentrationen lagen zwischen 0,01 % und 1 % (v/v) und waren bei den meisten der analysierten pflanzlichen Stoffe nicht zytotoxisch.

Jurkat-LAT-GFP-Zellen wurden 30 Minuten lang mit steigenden Konzentrationen der Testsubstanzen vorinkubiert und dann 12 Stunden mit entweder TNFα (2 ng/ml) oder PMA (10 ng/ml) stimuliert. Schließlich wurde die GFP-Expression durch Durchflusszytometrie in einem Durchflusszytometer vom Typ FACSCanto II analysiert. Es wurden zehntausend Ereignisse pro Probe gezählt und das Fluoreszenzmuster wurde bestimmt.

Die erfindungsgemässe Zusammensetzung reduzierte die Virusvermehrung in der Zellkultur. Bei einer Verdünnung der Zusammensetzung mit Wasser in einem Verhältnis von 1:10'000 wurde eine 1000fache Reduktion des Virustiters von HIV-1 beobachtet. Mit der Verdünnung von 1:30.000 erreichte die Reduktion noch immer den Faktor 300. Für HIV-2 war bei einer Verdünnung von 1:10.000 eine 30fache Titerreduktion zu beobachten.

Bestimmt wurde die Infektiosität einer Verdünnungsreihe von HIV-Viren durch Bewertung der Zellpathogenität:
- Vorlage von Jurkat-CD4-Zellen mit einer eingestellten Zellzahl (z.B. 6.000.000/Napf)
- Die Zusammensetzung wurde in einer Verdünnungsreihe im Medium vorbereitet. 500 µl der jeweiligen Verdünnung wurden zum Medium gegeben und inkubiert.
- 50 µl der Verdünnung des Referenzvirus wurden zur Zellkultur gegeben und inkubiert
- Medium und Zellen wurden durch Zentrifugation getrennt. Im Überstand wurde durch Standardtitrationstest der Virustiter bestimmt.
- Die Zellen selbst wurden in Medium mit Verdünnungen von V.II-BE über 28 inkubiert.
- Am Ende der Versuchsdauer wurde der zallpathogene Effekt bewertet.

Eine sechsstündige Inkubation mit der Zusammensetzung führte zu einer dosisabhängigen Inaktivierung von HIV. Bei einer Verdünnung von 1:10.000 im Medium im angegebenen Verhältnis reduzierte die Zubereitung die Infektiosität von HIV-1 um den Faktor 400.

### Versuch 8: Wirkung auf HIV-1 und HIV-2 Viren in einer Zellkultur

Bei einer Verdünnung der Zusammensetzung (erhalten nach Versuch 7) um 1:10.000 und bei einer Inkubationszeit von 6 h ergab sich eine 400fache Reduktion der HIV-1-Infektiosität in Jurkat-Zellen.

Bei einer Verdünnung der Zusammensetzung um 1:10.000 und bei einer Inkubationszeit von 6 h ergab sich eine 30-fache Reduktion der HIV-2-Infektiosität.

Bei einer Verdünnung der Zusammensetzung um 1:200 und bei einer Inkubationszeit von 6 h ergab sich eine 160fache Reduktion des Virustiters (HIV, nicht spezifiziert). Im Vergleich dazu ergab sich bei einer Kontrollprobe bei einer Verdünnung von 1:300 eine ca. 10-fache Reduktion des Virustiters, und bei einer Verdünnung von 1:1000 bis 10.000 eine solche von lediglich ca. 3.

Die Zusammensetzung reduzierte somit die Infektiosität von HIV-1 und HIV-2 in der Zellkultur in ganz erheblichem Masse.

### Versuch 9: Untersuchung der Toxizität der Zubereitung

Für die Untersuchung der chronischen Toxizität konnte die Zubereitung nicht direkt eingesetzt werden, weil die Alkoholkonzentration der dosislimitierende Faktor gewesen wäre. Aus diesem Grund wurde ein Pulver mit einer Zusammensetzung entsprechend der Mengenverhältnisse im Gesamtextrakt hergestellt. Dabei entsprachen die Mengenverhältnisse in der Pulvermischung denen der flüssigen Zusammensetzung. Dieses Pulver wurde dann per Schlundsonde an Wistar-Ratten oral verabreicht.

Im ersten Schritt wurde entsprechend der Regeln toxikologischer Studien eine Vorstudie über sieben Tage durchgeführt. In dieser Vorstudie kamen Dosen bis zu 1000 mg der Pulvermischung pro kg Körpergewicht zum Einsatz. Es wurden keine Hinweise auf Toxizität beobachtet.

Auf diese Vorstudie folgte die dreimonatige Toxizitätsstudie mit einer vierwöchigen Nachbeoachtungsdauer. Getestet wurden neben den makroskopischen Beobachtungen Laborparameter und die Histopathologie. Die Studie wurde in Einklang mit geltenden Bestimmungen für die Durchführung toxikologischer Studien mit drei Dosen durchgeführt: 25 mg/kg als niedrige Dosis, 75 mg/kg als mittlere Dosis, und 250 mg/kg als hohe Dosis.v Mit dieser Dosis wurden keinerlei Anzeichen von Toxizität beobachtet.

Aus den erhaltenen Befunden ist abzuleiten, dass auch bei absichtlicher Überdosierung, z.B. Einnahme des Inhaltes einer gesamten Flasche, toxische Effekte nicht zu erwarten sind.

### Versuch 9.: Synergistische Wirkung der Einzelkomponenten

Es wurde im Labor Einzelextrakte der sechs pflanzlichen Bestandteile hergestellt. Als Modell diente die TNF-α- oder PMA-induzierte Aktivierung von HIV in infizierten Jurkat-Zellen (gleiche Versuchsbedingungen wie weiter oben beschrieben).

Unter den Einzelstoffen zeigten sich dabei synergistische Verstärkungen zwischen den Bestandteilen Rose und Kamille sowie Rose und Lavendel.

### 1. Studie: Bestimmung HIV-Titer und CD4-Zellzahl

Untersucht wurde die Frage des Effektes der Zubereitung auf die Viruslast und die CD4-Zellzahl als Hauptparameter der HIV-Diagnostik. Die Patienten mit diagnostiziertem HIV hatten eine Viruslast < 50'000 Kopien/ml Blut und eine CD4+ - Zahl von durchschnittlich > 500 Zellen/□l Blut. Sie erhielten über einen Zeitraum von mindestens 60 bis 180 Tagen 2x täglich 60 Tropfen einer 0.5 prozentigen Zubereitung der Zusammensetzung nach Abdampfen des Extraktionsmittels.

In der Tabelle 1 sind die Daten dieser Patienten mit unterschiedlicher Expositionsdauer (bis zu vier Monate, in zwei Fällen sogar sechs Monate) graphisch dargestellt. Gemessen wurden neben HIV-Titern und CD4-Zellzahlen auch klinische Laborparameter als Maß der Anwendungssicherheit. Bei den Sicherheitsparameterm wurden keine unerwünschten Veränderungen beobachtet. Dagegen zeigen sich neben einer Verringerung der Viruslast (in vielen Fällen unter die Nachweisgrenze) durchwegs Anstiege der CD4-Zellzahl, was bei HIV-Patienten mit einer Verbesserung der Immunfunktion einhergeht (Tabelle 2).

Da in diese Studie nur Patienten mit vergleichsweise niedriger Viruslast (im Bereich der Nachweisgrenze) aufgenommen wurden, lag der Schwerpunkt der Auswertung auf dem Effekt der Zubereitung auf die CD4-Zellzahl.

Es wurde ein signifikanter Anstieg der CD4-Zellen um durchschnittlich 89,53 % errechnet (Tabelle 2). Die statistische Analyse erfolgte unter der Hypothese, dass es bei den Patienten, die nicht die Expositionsdauer von drei Monaten erreicht hatten, zu einer Verschlechterung der Werte gekommen sein könnte. Dabei ergab sich für die CD4-Zellzahl auch dann noch ein hochsignifikanter Wert, wenn es bei der unrealistisch hohen Zahl von 50% der dokumentierten Fälle zu einer Verschlechterung gekommen wäre. Die beobachteten Ergebnisse sind daher klinisch hochrelevant sowie statistisch signifikant und robust.

Die Anwendung der Zubereitung kann damit eine Therapielücke schließen: Bislang ist es nicht möglich, HIV-infizierten Patienten in Erkrankungsstadien ohne akute Immunschwächebeschwerden eine Hilfe zur Kontrolle der Viruslast und der Sicherstellung der Immunfunktion anzubieten.

### 2. Studie: Behandlung von Herpes labialis

Die durch HIV ausgelöste Immunschwäche begünstigt das Auftreten opportunistischer viraler Infekte wie Herpes labialis. In einer Falldatendokumentation wurde daher untersucht, ob die Verwendung der Zubereitung einen Einfluss auf den Verlauf von Eposiden von Herpes labialis haben kann.

Diese Falldatendokumentation (Tabelle 3) zeigt im Vergleich mit bibliographischen Werten eine gegenüber Aciclovir und Placebo statistisch signifikante und klinisch relevante Verkürzung der Dauer von Herpesepisoden um mindestens 2,5 bzw. 3,1 Tage (49,0 % bzw. 54,4 % Reduktion der Dauer der Abheilung bis zur Verkrustung).

Damit zeigte die Zubereitung auch antivirale Effekte gegen Herpes labialis.

Tabelle 4 zeigt, dass die erfindungsgemässe Zusammensetzung dosisabhängig die TNFα - induzierte HIV-LTR Transaktivierung in Jurkat 5.1 - Zellen hemmt.

Weitere Studien, die die Erfinderin hat durchführen lassen, zeigen, dass ein Extrakt der stechenden Binse überraschenderweise für sich allein die HIV - Tat (trans-activator of transcription) induzierte LTR Transaktivierung hemmt. Dies ist eine neue Erkenntnis, die bislang in der Literatur noch nicht dokumentiert ist. Auf der anderen Seite wurde festgestellt, dass die Stechende Binse die MAPK - Signalwege nicht aktiviert und die PMA-induzierte ERK und JNK (MAPK) Aktivierung hemmt.

The kombinierte anti-Tat - (herrührend von der stechenden Binse) und anti-NFκB Aktivitäten (vermutlich auf die Rosenblüten- und Weidenextrakte zurückführend) könnte die in vitro festgestellte potente anti - HIV - 1 Aktivität der erfindungsgemässen Zusammensetzung erklären.

Weitere gefundene Ergebnisse sind:
Extrakte der Myrrhe, Rosenblüten und Weidenrinde aktivierten den JNK Signalweg.
Extrakte der Myrrhe, Rosenblüten und Lavendel aktivierten den ERK Signalweg.
Rosenblüten- und Kamilleextrakte verstärkten den HIV - Tat induzierte LTR-Aktivierung.

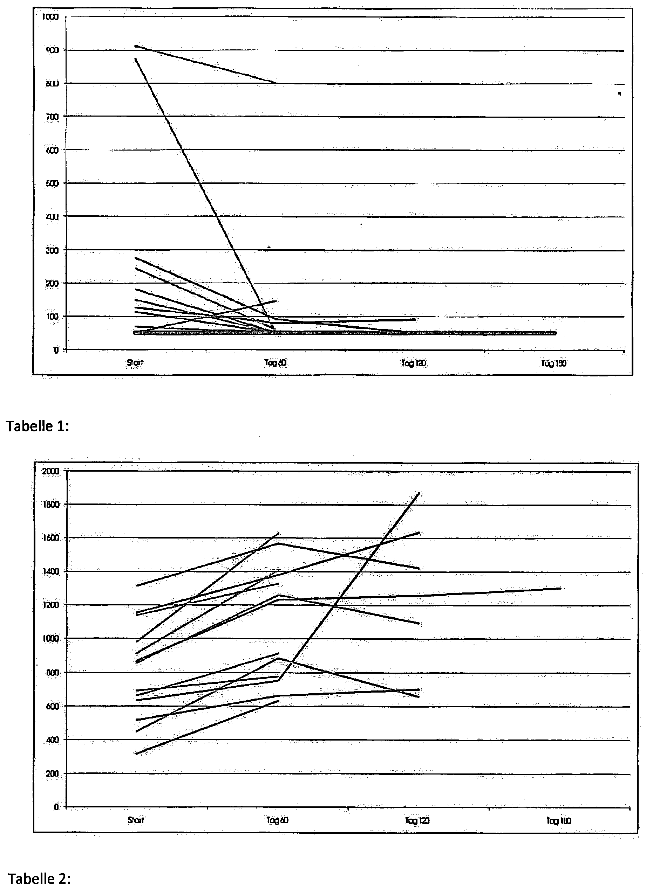

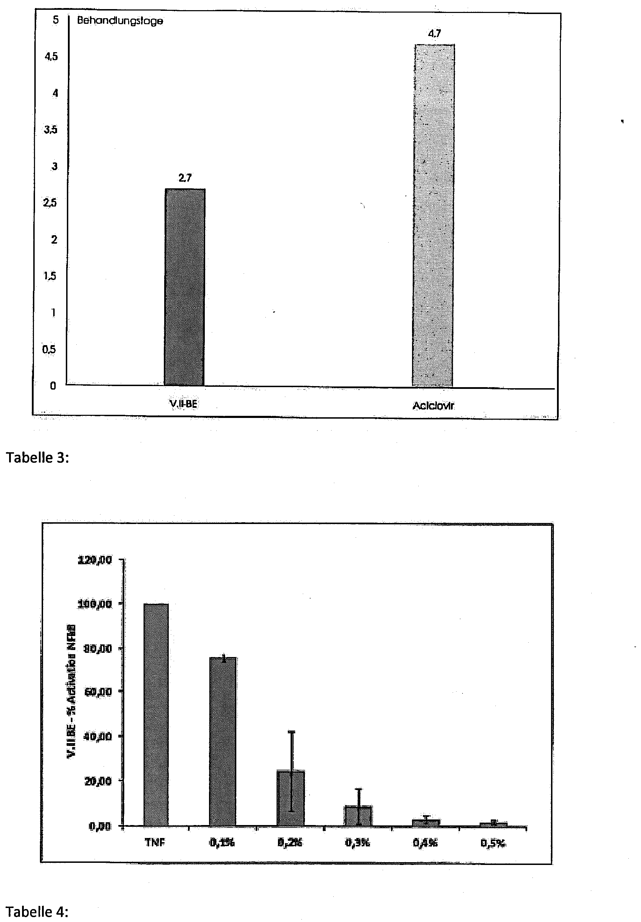

## Patentansprüche

1. Pharmazeutische Zusammensetzung geeignet zur Verwendung als antivirale Zusammensetzung oder Virostatikum, enthaltend Extrakte der Pflanzenart Stechende Binse und der Pflanzengattungen Rose, Kamille, Lavendel, Silberweide und Myrrhe als wirksame Bestandteile.

2. Pharmazeutische Zusammensetzung nach Anspruch 1, **dadurch gekennzeichnet, dass** von den Extrakten der Pflanzengattung "Silberweide" Extrakte der Pflanzenart "Salix alba", von den Extrakten der Pflanzengattung "Rose" Extrakte der Pflanzenart "Rosa damascena", von den Extrakten der Pflanzengattung "Kamille" Extrakte der Pflanzenart "Chamomilla matricaria", von den Extrakten der Pflanzengattung "Lavendel" Extrakte der Pflanzenart "Lavandula angustifolia" und von den Extrakten der Pflanzengattung "Myrrhe" Extrakte der Pflanzenart "Commiphora molmol" eingesetzt sind.

3. Pharmazeutische Zusammensetzung nach Anspruch 1oder 2 erhältlich durch Extraktion mit einer wässerigen Alkoholmischung enthaltend wenigstens 70 Gew.-% und besonders bevorzugt wenigstens 85 Gew.-%, Alkohol, wobei als Alkohol vorzugsweise Methanol, Ethanol oder Propanol, oder eine Mischung der vorgenannten Alkohole verwendet ist.

4. Pharmazeutische Zusammensetzung nach Anspruch 1 oder 2, erhältlich durch Extraktion der nachfolgend genannten Mengen Ausgangsprodukt, jeweils als Trockenware:
| | |
|---|---|
| Kamillenblüten (Blütenstand mit Korb und Blüte) | 4.00 Kg |
| Rosenblüten (Rosa damascena) | 8.00 Kg |
| Lavendelblüten | 1.20 Kg |
| Myrrhe (Harz) | 0.07 Kg |
| Binsengras (Juncus acutus) | 3.00 Kg |
| Weidenrinde | 0.80Kg |
mit einer wässerigen, wenigstens 80% Ethanol enthaltenden Alkoholmischung und Verdünnen des alkoholischen Extrakts mit Wasser auf 200 Litern und 0,1 bis 1,0 % Alkoholgehalt.

5. Pharmazeutische Zusammensetzung nach einem der Ansprüche 1 bis 4, **dadurch gekennzeichnet, dass** Zusammensetzung in einer Verdünnung, vorzugsweise mit Wasser, zwischen 0.001 % bis 10 % (w/w), vorzugsweise zwischen 0.01 % bis 1 % (w/w), und besonders bevorzugt zwischen 0.1 % bis 0.5% (w/w) eingesetzt wird.

6. Pharmazeutische Zusammensetzung gemäss einem der Ansprüche 1 bis 5 zur Verwendung als Prophylaxe oder Bekämpfung von Herpes labialis, Herpes genitalis oder Herpes zoster Infektionen.

7. Pharmazeutische Zusammensetzung gemäss einem der Ansprüche 1 bis 5 zur Verwendung als Prophylaxe oder Bekämpfung von Grippeviren (Influenza).

8. Pharmazeutische Zusammensetzung gemäss einem der Ansprüche 1 bis 5 zur Verwendung als Prophylaxe oder Bekämpfung von Erkältungskrankheiten, deren Ursache viraler Natur sind.

9. Pharmazeutische Zusammensetzung gemäss einem der Ansprüche 1 bis 5 zur Verwendung als Prophylaxe oder Bekämpfung von HIV Infektionen.

## Claims

1. Pharmaceutical composition suitable for use as antiviral composition or antiviral containing extracts of the plant species spiny rush (juncus acutus) or of the plant genera rose, chamomile, lavender, white willow and myrrh as effective ingredients.

2. Pharmaceutical composition according to claim 1, **characterized in that** among the extracts of the plant genus white willow extracts of the plant species "salix albas", among the extracts of the plant genus "rose" extracts of the plant species "rosa damascena", among the extracts of the plant species "chamomile" extracts of the plant species "chamomilla matricaria", among the extracts of the plant genus "lavender" extracts of the plant species "lavandula angustifolia" and among the extracts of the plant genus "myrrh" extracts of the plant species "commiphora molmol" are used.

3. Pharmaceutical composition according to claim 1 or 2 obtained by extraction with an aqueous alcohol mixture containing at least 70% by weight and particularly preferably at least 85% by weight alcohol wherein preferably methanol, ethanol or propanol is used as alcohol or a mixture of aforesaid alcohols.

4. Pharmaceutical composition according to claim 1 or 2 obtained by extraction of the quantities of starting product listed below, respectively as dry good:
| | |
|---|---|
| Chamomile blossoms (inflorescence withheadand blossom) | 4 kg |
| Rose blossoms (rosa damascena) | 8 kg |
| Lavender flowers | 1,20 kg |
| Myrrh (resin) | 0,07 kg |
| Rush (juncus acutus) | 3 kg |
| Willow bark | 0,8 kg |
with an aqueous alcohol mixture containing at least 80% ethanol and dilution of the alcoholic extract with water in 200 liter and 0,1 to 1,0% alcohol content.

5. Pharmaceutical composition according to one of the claims 1 to 4, **characterized in that** the composition is used in a dilution, preferably with water, between 0,001% to 10% (w/w), preferably between 0,01% to 1 % (w/w) and particularly preferably between 0,1% to 0,5% (w/w).

6. Pharmaceutical composition according to one of the claims 1 to 5 for use as prophylaxis or for controlling Herpes labialis, Herpes genitalis or Herpes zoster infections.

7. Pharmaceutical composition according to one of the claims 1 to 5 for use as prophylaxis or for controlling flue viruses (influenza).

8. Pharmaceutical composition according to one of the claims 1 to 5 for use as prophylaxis or for controlling common colds with viral cause.

9. Pharmaceutical composition according to one of the claims 1 to 5 for use as prophylaxis or for controlling HIV infections.

## Revendications

1. Composition pharmaceutique appropriée pour l'usage comme composition antivirale ou antiviral contenant des extraits de l'espèce végétale jonc piquant (juncus acutus) ou des espèces rose, camomille, lavande, saule et myrrhe comme principes actifs.

2. Composition pharmaceutique selon la revendication 1, **caractérisée en ce que** pour les extraits de l'espèce végétale du saule, des extraits de l'espèce "salix alba", pour les extraits de l'espèce végétale de la rose, des extraits de l'espèce "rosa damascena", pour les extraits de l'espèce végétale de la camomille, des extraits de l'espèce "chamomilla matricaria", pour les extraits de l'espèce végétale de la lavande, des extraits de l'espèce "lavandula angustifolia" et pour les extraits de l'espèce végétale de la myrrhe des extraits de l'espèce "commiphora molmol" sont utilisés.

3. Composition pharmaceutique selon la revendication 1 ou 2 que l'on obtient par extraction avec un mélange aqueux d'alcool qui contient au moins 70% en poids d'alcool, et de manière particulièrement préférée au moins 85% en poids, cependant que comme alcool on utilise de préférence du méthanol, de l'éthanol ou du propanol ou un mélange des alcools sus-cités.

4. Composition pharmaceutique selon la revendication 1 ou 2 que l'on obtient par extraction des quantités ci-dessous de produit de départ, chacune comme produit sec :
| | |
|---|---|
| Fleurs de camomille (inflorescence avec capitule et fleur) | 4 kg |
| Fleurs de rose (rosa damascena) | 8 kg |
| Fleurs de lavande | 1,20 kg |
| Myrrhe (résine) | 0,07 kg |
| Jonc (juncus acutus) | 3 kg |
| Ecorce de saule | 0,8 kg |
avec un mélange aqueux d'alcool qui contient au moins 80% d'éthanol et dilution de l'extrait alcoolique avec de l'eau sur 200 litres et 0,1 à 1% de teneur en alcool.

5. Composition pharmaceutique selon l'une des revendications 1 à 4, **caractérisée en ce que** la composition est mise en place dans une dilution, de préférence avec de l'eau, entre 0,001% à 10% (poids/poids), de préférence entre 0,01% à 1% (poids/poids) et de manière particulièrement préférée entre 0,1% et 0,5% (poids/poids).

6. Composition pharmaceutique selon l'une des revendications 1 à 5 pour l'utilisation comme traitement préventif ou pour traiter des infections causées par l'herpès comme l'herpès labial, l'herpès génital ou le zona.

7. Composition pharmaceutique selon l'une des revendications 1 à 5 pour l'utilisation comme traitement préventif ou pour traiter des virus grippaux (grippe).

8. Composition pharmaceutique selon l'une des revendications 1 à 5 pour l'utilisation comme traitement préventif ou pour traiter des rhumes dont la cause est d'origine virale.

9. Composition pharmaceutique selon l'une des revendications 1 à 5 pour l'utilisation comme traitement préventif ou pour traiter des infections au VIH.
